Europäisches Patentamt

(19) European Patent Office

Office européen des brevets

(11) Publication number: 0 268 316
A1

(12) EUROPEAN PATENT APPLICATION

(21) Application number: 87202049.0

(22) Date of filing: 26.10.87

(51) Int. Cl.4: A61L 9/12

(30) Priority: 27.10.86 NL 8602683

(43) Date of publication of application:
25.05.88 Bulletin 88/21

(84) Designated Contracting States:
BE DE FR NL

(71) Applicant: Ten Klei Specials B.V.
Molenkade 44
NL-1115 ZG Duivendrecht(NL)

(72) Inventor: Ten Klei, Johannes R.
Burgemeester D. Kooimanweg 100
NL-1444 DB Purmerend(NL)

(74) Representative: Smulders, Theodorus A.H.J.
et al
Vereenigde Octrooibureaux Nieuwe Parklaan
107
NL-2587 BP 's-Gravenhage(NL)

(54) A method of making a strippable material containing odorant, and intermediate product produced by applying part of said method.

(57) The invention relates to a method of making a strippable material containing odorant, wherein a slurry of microcapsules bearing an odorant therein and adhesive is applied to a flexible carrier, which is then folded and dried, wherein the slurry is applied in the direction of advance of the carrier mirror symmetrically on both sides of a longitudinal axis, after which the applied slurry is first dried prior to folding the carrier around its longitudinal axis and then thermally activating the dried slurry, in such a way that the now contiguous sections formed from the applied and dried slurry are bonded together in an easily strippable fashion.

EP 0 268 316 A1

# A method of making a strippable material containing odorant, and intermediate product produced by applying part of said method.

This invention relates to a method of making a strippable material containing odorant, wherein a slurry of microcapsules bearing an odorant therein and adhesive is applied to a flexible carrier which is then folded and dried.

Such a method is known from U.S. patent 4,487,801 and from European patent application 0 189 656. According to the method disclosed in these two publications the slurry is applied in the form of a continuous line in the longitudinal direction of a web-like carrier, preferably a paper web, near its central portion, after which the web-like carrier is folded in the longitudinal direction and is dried. Such a procedure, in which the strip layer is a single layer, is difficult to control in such a way as to ensure efficient separation of the strip layer upon subsequent use of the material. In fact, this requires fulfilment of critical conditions with respect to the bonding strength between carrier and strip layer, the inherent strength of the strip layer, the rupture strength of the capsules and the sizes of these capsules, as exhaustively discussed in the above-mentioned publications. This prior art method is therefore laborious and expensive, and the quality of the final product is inconstant.

The object of this invention is to provide a simple and cheap method of making a strippable material which is very reliable in use.

This object is achieved, according to the invention, by conducting the method described in the opening paragraph in such a way that the slurry is applied in the direction of advance of the carrier mirror symmetrically on both sides of a longitudinal axis, after which the slurry applied is first dried prior to folding the carrier around its longitudinal axis and then thermally activating the dried slurry, in such a manner that the now contiguous sections formed from the applied and dried slurry are bonded together in an easily strippable fashion.

The essential difference with the prior art as previously described therefore resides in that the slurry is applied, in the manner described, on both sides of the longitudinal axis and is first dried so as to obtain a strong bond to the carrier, after which, in the folded material, the contiguous sections of dried slurry are readily adhered together. It is clear that in such a procedure some conditions are much less critical than according to the prior art, so that the entire manufacturing process is simpler and cheaper and moreover, results in a final product which is more reliable in use.

According to the invention, therefore, the strip layer is, in fact, composed of two layers which are bonded together only through an easily rupturable bond. Upon layer stripping, a number of capsules are ruptured, so that the odorant is released. For instance, by rubbing the strip or rupture surfaces with a finger other capsules can be ruptured, so that odorant is released again.

According to a preferred embodiment the slurry is applied in sections which are separated from each other in the direction of advance of the carrier. In such a procedure the individual odorant sections are properly screened on all sides with no vulnerable edge portion where odorant is easily released unintentionally. According to a further favorable form of the above-described preferred embodiment different odorants are included in at least two sections following each other in the direction of advance of the carrier. Thus a number of odorants can be combined to form one final product, in which the separate release of those odorants can be facilitated by providing the carrier between successive odorant sections with a perforation extending from the longitudinal axis to one of the side edges of the carrier.

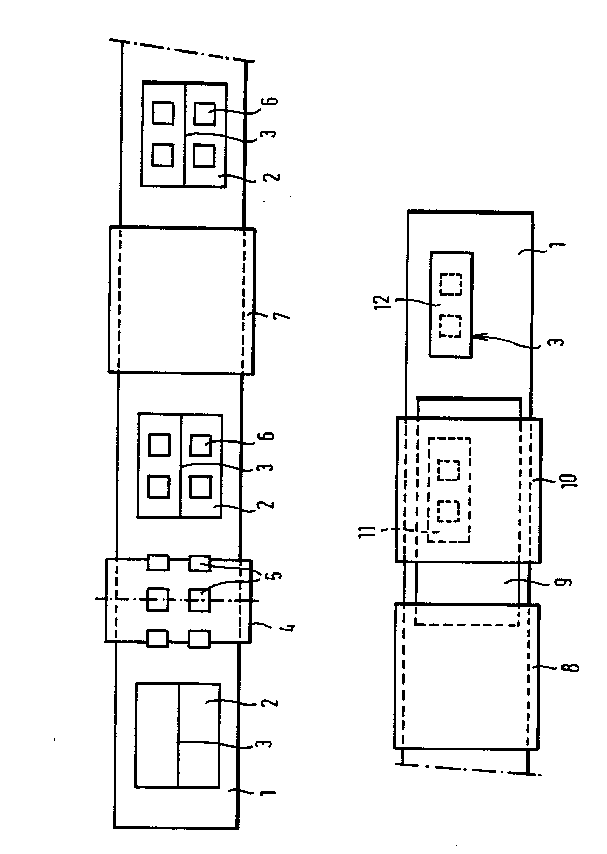

This invention will now be further elucidated, partly with reference to the only figure of the drawing, which figure is a diagrammatic representation of the manufacturing process. The figure shows a conveyor belt 1 provided with a sheet-like carrier 2 having a folded seam 3. This carrier may consist of very different flexible materials but is preferably a sheet of ordinary paper, on which for the present use no special demands are imposed. The conveyor belt then passes an application roll 4 provided with application elements 5 for pressing the slurry on the carrier 2. The application elements 5 may consist of, e.g., a slightly spongy material capable of absorbing the slurry and then depositing same on the carrier 2. The carrier 2 provided with the pressed-on slurries 6 is then passed to a drier 7, where the pressed-on slurries 6 are dried and firmly connected to the carrier material. For the drying process any suitable drying technique may be used, such as high-frequency drying, infrared drying, drying with a hot air stream etc. After drying, the now dried odorant sections 6 are located on the carrier 2. The resulting material can be stored as an intermediate product or sold for further processing, which, however, often takes place immediately afterwards. The carrier is then folded around the folded seam 3 in a folder 8, after which the folded carrier 11 enclosing the now contiguous pressed-on slurries 6 is gently held together by a band 9, e.g., made of teflon, and passed through a heater 10 for effecting the seam joint which, at a later time, is easy to rupture by stripping. Also here various heat supplying techniques can be used, such as those previously mentioned for use in the drier. In any case the temperature and the heating time can be easily controlled as a function of the composition of the applied slurry and of the desired bonding strength rupturable by stripping. Eventually, a final product 12 is obtained which, if desired, can be cut into the desired formats and/or can be packed, etc.

The slurry to be used in this process can be prepared according to simple and known per se techniques. A desired odorant, e.g., a fragrant oil, can be encapsulated in microcapsules having sizes of, e.g., 10-30$\mu$m. A slurry of these capsules is then mixed in a mixing ratio of, e.g. 1/1.5-2.5 with an adhesive in liquid form. It is important that the adhesive should be thermally activatable, in other words: should have thermoplastic properties, and in any case the solid substance of the adhesive and/or the material of the capsule wall must have a base of thermoplastic substance or consist thereof, because otherwise no thermal compound can be effected between the contiguous dried pressed-on slurries. Suitable thermoplastic substances are generally known in the art and may be selected from, e.g., vinyl and vinylidene polymers, etc. Of course, it is also conceivable that the dried pressed-on slurries are interconnected via separate adhesive layer or that the bond is effected by wetting the dried pressed-on slurries again, when using a water-activatable adhesive, but such embodiments require an additional treatment step and are therefore not eligible.

It is self-evident that a number of variants of the manufacturing process previously described in detail with reference to the figure are simply possible without departing from the scope of this invention.

Accordingly, instead of the sheet-like carrier 2, there may also be used an uncoiled band-like carrier, which may also be pressed through the successive treatment stations with auxiliary devices other than the conveyor belt 1 as shown. Also, the folded seam 3 may be applied only a later stage, namely after drying in the drier 7 or in folder 8 itself. Instead of only one application roll 4, there may also be used a number of application rolls. Indeed, the application of the slurry is not limited to the technique as shown using a roll 4 provided with application elements 5. In principle, any suitable technique may be used, such as the application via a slot or with a doctor. The geometric form of the pressed-on slurries 6 may vary too. Instead of the square form as shown, in fact, any other desired form may be effected by pressing on. Finally, the pressed-on slurries need not necessarily form sections separated from each other in the direction of advance of the carrier, as shown in the figure, which, however, is preferred for the reasons previously mentioned, but according to a less preferred embodiment a continuous, i.e. and uninterrupted pressed-on slurry may be also applied in the direction of advance of the carrier.

Finally, all kinds of desirable texts or figures may be applied to the carrier, e.g. by means of conventional printing techniques. Also, the carrier may be provided on the outer surface with an adhesive layer, to which a temporary, and strippable cover sheet may be applied, so that the final product is a sticker. These embodiments are of course quite unconnected with the essence of the invention.

## Claims

1. A method of making a strippable material containing odorant, wherein a slurry of microcapsules bearing an odorant therein and adhesive is applied to a flexible carrier which is then folded and dried, characterized in that the slurry is applied in the direction of advance of the carrier mirror symmetrically on both sides of longitudinal axis, after which the applied slurry is first dried prior to folding the carrier around its longitudinal axis and then thermally activating the dried slurry, in such a way that the now contiguous sections formed from the applied and dried slurry are bonded together in an easily strippable fashion.

2. A method according to claim 1, characterized in that the slurry is applied in sections which are separated from each other in the direction of advance of the carrier.

3. A method according to claim 2, characterized in that at least two sections following each other in the direction of advance of the carrier contain a different odorant.

4. A method according to claims 1-3, characterized in that the slurry is applied on both sides of the longitudinal axis at some distance from said axis.

5. An intermediate product obtained by applying the method according to one of claims 1-4 up to and including the drying step.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 487 801  (TURNBULL)<br>* Example 1 *<br>--- | 1,2 | A 61 L   9/12 |
| A,D | EP-A-0 189 656  (MINNESOTA MINING AND MANUFACTURING CO.)<br>* Page 4, lines 6-10; page 11, lines 6-11 *<br>--- | 1,2 | |
| A | DE-A-3 415 156  (H. BREM)<br>* Claims *<br>--- | 3 | |
| A | GB-A-1 273 322  (NATIONAL CASH REGISTER CO.)<br>* Example 1 *<br>----- | | |

TECHNICAL FIELDS
SEARCHED (Int. Cl.4)

A 61 L

The present search report has been drawn up for all claims

| Place of search | Date of completion of the search | Examiner |
|---|---|---|
| THE HAGUE | 04-02-1988 | COUSINS-VAN STEEN G.I.L. |

EPO FORM 1503 03.82 (P0401)